# EUROPEAN PATENT APPLICATION

(11) **EP 4 817 591 A1**
(43) Date of publication of application: **07.10.2026**
(21) Application number: 25766866.5
(22) Date of filing: 30.07.2025
(51) Int. Cl.: A61K 8/19, A61K 8/25, A61K 8/26, A61Q 11/00

(54) **TOOTHPASTE**

(30) Priority: 26.01.2025 CN 202510125178
(71) Applicant: Olympic Sports Twelvefold Crowns Health Development (Hangzhou) Co., Ltd., Hangzhou, Zhejiang 310013 (CN)
(72) Inventor: QU, Yingliang, Hangzhou, Zhejiang 310013 (CN); JIN, Zhe, Hangzhou, Zhejiang 310013 (CN); HUANG, Guangming, Hangzhou, Zhejiang 310013 (CN)
(74) Representative: Petraz, Gilberto Luigi
(86) International application number: PCT/CN2025/111496
(87) International publication number: WO 2026/157169

(57) **Abstract**

A toothpaste is provided. The toothpaste at least includes 5% to 20% of silicon dioxide and 5% to 15% of zeolite by a mass fraction. An average particle size of the silicon dioxide is defined as *R₁*, a Brinell hardness of the silicon dioxide is defined as *H₁*, an average particle size of the zeolite is defined as *R₂*, and a Brinell hardness of the zeolite is defined as *H₂*. *R₁*, *R₂*, *H₁*, *H₂* meet following relationships: 20 µm ≤ *R₁* ≤ 40 µm, 1 µm ≤ *R₂* ≤ 10 µm, 5 ≤ *R₁* / *R₂* ≤ 20, 2.5 HB ≤ *H₁* ≤ 3.5 HB, 4.0 HB ≤ *H₂* ≤ 6HB, and 1.3 ≤ *H₂* / *H₁* ≤ 2.

## Description

### CROSS-REFERENCE TO RELATED APPLICATION

This application claims priority to Chinese patent application No. 202510125178.7, filed on January 26, 2025, and titled "TOOTHPASTE". The content of the above identified application is hereby incorporated herein in its entirety by reference.

### TECHNICAL FIELD

The present application relates to the field of oral care technology, and in particular, to a toothpaste.

### BACKGROUND

As an oral cleaning product, toothpaste is used to prevent cavities and maintain fresh breath. Main components of the toothpaste include a friction agent, a humectant, a surfactant and so on. A friction agent as an important component of the toothpaste, is mainly used for removing dirt and plaque on surfaces of teeth and has an effect of whitening the teeth. However, if a friction force of the friction agent is poor, the effect of whitening the teeth is not obvious, and the cleaning effect is poor; if the friction force of the friction agent is too strong, the tooth surfaces and periodontal tissues may be damaged. In addition, diseases such as gingivitis and periodontitis can cause gum inflammation, and bleeding can occur after touching. Meanwhile, as the teeth is brushed, gingivitis may cause bleeding due to excessive force or hard tooth bristles, although most related toothpastes adopt western medicine components for hemostasis.

### SUMMARY

According to various embodiments of the present application, a toothpaste is provided.

A toothpaste at least includes 5% to 20% of silicon dioxide and 5% to 15% of zeolite by mass fraction. An average particle size of the silicon dioxide is defined as *R₁*. A Brinell hardness of the silicon dioxide is defined as *H₁*. An average particle size of the zeolite is defined as *R₂*. A Brinell hardness of the zeolite is defined as *H₂*. The average particle size *R₁* of the silicon dioxide meets the following relationship: 20 µm ≤ *R₁* ≤ 40 µm. The average particle size R₂ of the zeolite meets the following relationship: 1 µm ≤ *R₂* ≤ 10 µm. The average particle size *R₁* of the silicon dioxide and the average particle size *R₂* of the zeolite meet the following relationship: 5 ≤ *R₁* / *R₂* ≤ 20. The Brinell hardness *H₁* of the silicon dioxide meets the following relationship: 2.5 HB ≤ *H₁* ≤ 3.5 HB. The Brinell hardness *H₂* of the zeolite meets the following relationship: 4.0 HB ≤ *H₂* ≤ 6HB. The Brinell hardness *H₁* of the silicon dioxide and the Brinell hardness *H₂* of the zeolite meet the following relationship: 1.3 ≤ *H₂* / *H₁* ≤ 2.

In an embodiment, the Brinell hardness *H₂* meets the following relationship: 4.0 HB ≤ *H₂* ≤ 5.5 HB.

In an embodiment, the mass fraction of the silicon dioxide in the toothpaste is less than or equal to that of the zeolite in the toothpaste.

In an embodiment, a mass ratio of the silicon dioxide to the zeolite is in a range of 1: 1 to 1: 3.

In an embodiment, a specific surface area of the zeolite is greater than or equal to 50 m²/g⁻¹. Alternatively, a pore size of the zeolite is greater than or equal to 0.5 nm.

In an embodiment, by a mass fraction of calcium ions in the zeolite is in a range of 0.5% to 3%.

In an embodiment, strontium ions are loaded on the zeolite.

In an embodiment, the average particle size *R₁* of the silicon dioxide meets the following relationship: 20 µm ≤ *R₁* ≤ 30 µm, the average particle size *R₂* of the zeolite meets the following relationship: 5 µm ≤ *R₂* ≤ 10 µm, and the Brinell hardness *H₁* of the silicon dioxide and the Brinell hardness *H₂* of the zeolite meet the following relationship: 1.5 ≤ *H₂* / *H₁* ≤ 2.

In an embodiment, a mass fraction of the strontium ions in the zeolite is in a range of 0.2% to 2.0%.

In an embodiment, a mass ratio of the strontium ions in the zeolite to the calcium ion in the zeolite is in a range of 1: 1 to 4: 1.

Details of one or more embodiments of this application are presented in the attached drawings and descriptions below. And other features, purposes and advantages of this application will become apparent from the description, drawings and claims.

### DETAILED DESCRIPTION

To facilitate understanding of the present application, the disclosure is described in more detail below. However, it should be understood that the present application may be implemented in many different forms and is not limited to implementations or embodiments described herein. Conversely, the embodiments or the examples are provided to provide a more thorough and comprehensive understanding of the present application.

Unless otherwise defined, all technical and scientific terms used in the present application have the same meanings as those commonly understood by the skilled in the technical field of the present application. The terms used in the specification of the present application are only for a purpose of describing specific embodiments or examples and are not intended to limit the present application. Alternative ranges of the term "and/or" used in the present application include any one of two or more related listed items, and any and all combinations of related listed items includes any two of the related listed items, any more of the related listed items, or all of the related listed items.

In the present application, a numerical interval is referred to, unless otherwise specified, the numerical interval is considered continuous and includes a minimum value and a maximum value of the range, and each value between the minimum value and the maximum value. Furthermore, when the range refers to an integer, each integer between the minimum value and the maximum value of the range is included. Furthermore, when multiple ranges are provided to describe features or characteristics, a plurality of ranges may be merged. In other words, unless otherwise indicated, all ranges disclosed herein should be understood to include any and all subranges subsumed therein.

At present, common friction agents of the toothpaste include silicon dioxide (i.e., hydrated silica), calcium carbonate, calcium hydrogen phosphate, calcium pyrophosphate or aluminum hydroxide. The friction agents each has different friction effects due to particle size, hardness and so on of the friction agents. In order to ensure a cleaning effect of the toothpaste without damaging the teeth due to transitional friction, the toothpaste of the present application at least includes silicon dioxide and zeolite. The silicon dioxide and the zeolite are compounded as a friction agent. Hardness of the silicon dioxide is generally lower than that of the zeolite, and in combination with characteristics of the silicon dioxide and zeolite, the toothpaste of the present application meets the following conditions: a mass fraction of the silicon dioxide in the toothpaste is in a range of 5% to 20%, such as 5%, 6%, 7%, 8%, 9%, 10%, 11%, 12%, 13%, 14%, 15%, 16%, 17%, 18%, 19%, 20%, and so on. An average particle size *R₁* of the silicon dioxide is in a range of 20 µm to 40 µm, such as 20 µm, 21 µm, 22 µm, 23 µm, 24 µm, 25 µm, 26 µm, 27 µm, 28 µm, 29 µm, 30 µm, 31 µm, 32 µm, 33 µm, 34 µm, 35 µm, 36 µm, 37 µm, 38 µm, 39 µm, 40 µm, and so on. A Brinell hardness *H₁* of the silicon dioxide is in a range of 2.5 HB to 3.5 HB, such as 2.5 HB, 2.6 HB, 2.7 HB, 2.8 HB, 2.9 HB, 3.0 HB, 3.1 HB, 3.2 HB, 3.3 HB, 3.3 HB, 3.4 HB, 3.5 HB and so on.

A mass fraction of the zeolite in the toothpaste is in a range of 5% to 15%, such as 5%, 6%, 7%, 8%, 9%, 10%, 11%, 12%, 13%, 14%, 15%, and so on. An average particle size *R₂* of the zeolite is in a range of 1 µm to 10 µm, such as 1 µm, 2 µm, 3 µm, 4 µm, 5 µm, 6 µm, 7 µm, 8 µm, 9 µm, 10 µm, and so on. A Brinell hardness *H₂* of the zeolite is in a range of 4.0 HB to 6.0 HB, such as 4.0 HB, 4.1 HB, 4.2 HB, 4.3 HB, 4.4 HB, 4.5 HB, 4.6 HB, 4.7 HB, 4.8 HB, 4.9 HB, 5.0 HB, 5.1 HB, 5.2 HB, 5.3 HB, 5.4 HB, 5.5 HB, 5.6 HB, 5.7 HB, 5.8 HB, 5.9 HB, 6.0 HB and so on.

Meanwhile, the particle size ratio *R₁* / *R₂* of the silicon dioxide to the zeolite is further in a range of 5 to 20, for example, the particle size ratio *R₁* / *R₂* can be 5, 6, 7, 8, 9, 10, 11, 12, 13, 14, 15, 16, 17, 18, 19, 20, and so on. A Brinell hardness ratio *H₂* / *H₁* of the zeolite to the silicon dioxide is in a range of 1.3 to 2, such as 1.3, 1.4, 1.5, 1.6, 1.7, 1.8, 1.9, 2, and so on.

Therefore, the silicon dioxide and the zeolite are compounded to serve as the friction agent of the toothpaste, and the particle sizes, the Brinell hardness and the contents of the silicon dioxide and the zeolite are adjusted and worked in synergy. When the toothpaste uses, the silicon dioxide drives the zeolite to roll and generate mechanical friction on surfaces of the teeth, which is similar to that a large gear drives a small gear to roll instead of independent friction. Therefore, the cleaning effect can be ensured, and the teeth can be prevented from being damaged due to transition friction.

Furthermore, the zeolite has excellent adsorption and hemostasis performance, and adsorption, wound healing promotion and so on can assist hemostasis. When the zeolite is used as a friction agent for the toothpaste, friction of the silicon dioxide can better assist hemostasis.

Furthermore, when the Brinell hardness *H₂* of the zeolite is controlled to be in a range of 4.0 HB to 5.5 HB, facilitating avoiding damage to the teeth due to the transitional friction.

In the toothpaste of the present application, the average particle size *R₁* of the silicon dioxide is relatively great, the average particle size *R₂* of the zeolite is relatively small. In some embodiments, in combination with characteristics of the Brinell hardness of the silicon dioxide and the zeolite, when the mass ratio of the zeolite in the toothpaste is greater than that of the silicon dioxide in the toothpaste, the silicon dioxide can better drive the zeolite to roll on the tooth surface and play a better machinery friction role during use. Furthermore, in some embodiments, the mass ratio of the silicon dioxide to the zeolite is in a range of 1: 1 to 1: 3, such as 1: 1, 1: 1.5, 1: 2, 1: 2.5, 1: 3, and so on.

Furthermore, in some embodiments, a specific surface area of the zeolite is greater than or equal to 50 m²/g. Alternatively, in some embodiments, a pore size of the zeolite is greater than or equal to 0.5 nm, so that the zeolite can better adsorb dental plaque, pigment, peculiar smell and so on which are located on the surfaces of the teeth, facilitating cleaning the surfaces of the teeth, maintaining oral hygiene, and meanwhile better assisting in the hemostasis.

When the zeolite is used for assisting the hemostasis, the zeolite is related to a concentration of released calcium ions, in the present application, since the silicon dioxide can drive the zeolite to roll and generate mechanical friction, in some embodiments, a mass fraction of calcium ions in the zeolite is in a range of 0.5% to 3%, such as 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, and so on. The mass fraction of the calcium ions in the oral environment is in such range , facilitating the hemostasis.

Dental allergy refers to pain caused by stimuli such as temperature, mechanical, chemical, and so on when enamel of the tooth (also known as French) gradually wears to expose a dentin, for example, the stimuli may be generated when food such as high sugar content, sour fruit and so on is eaten, or cold water is drunk. At present, strontium salts and so on are generally applied in toothpaste as a desensitization agent. However, when a content of the desensitization agent such as the strontium salts in the toothpaste is high, the toothpaste has poor taste during use.

In the present application, furthermore, in some embodiments, the zeolite is further loaded with strontium ions. Since during using, the silicon dioxide can drive the zeolite to roll and generate the mechanical friction on the surfaces of the teeth, and the zeolite of the present application can dynamically and continuously release the strontium ions, such that the concentration of the strontium ions in an oral environment is in a relatively stable state, which not only can avoid a problem of poor taste caused by too high concentration of the strontium ions, i.e., improve a taste of the toothpaste, but also can ensure that the strontium ions can be continuously and stably adsorbed to the enamel and the dentin, generate insoluble salt strontium carbonate (SrCO₃) by reacting with carbonate, and generate hydroxyapatite (Ca₃ (PO₄)Sr(OH)₂) by reacting with hydroxyapatite of dentin, which has effects of sealing the tubules and mineralizing the dentin, thereby slowing a conduction of stimulation and improving an anti-allergic ability of the teeth. It should be noted that above processes are continuous and stable, resulting in a better anti-allergic effect.

In order that the zeolite is driven by the silicon dioxide to rapidly roll on the surfaces of the teeth, effectively rub and clean, and the strontium ions is released dynamically and continuously, and the strontium ions in the oral environment are adsorbed by dentin and seal tubules within a short time in a process of brushing the teeth, in some embodiments, the average particle size *R₁* of the silicon dioxide is in a range of 20 µm to 30 µm, the average particle size *R₂* of the zeolite is in a range of 5 µm to 10 µm, and the ratio of the Brinell hardness *H₂* of the zeolite to the Brinell hardness *H₁* of the silicon dioxide is in a range of 1.5 to 2.

Furthermore, the mass fraction of strontium ions in the zeolite is in a range of 0.2% to 2.0%, such as 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, and so on, which can better control the concentration of the strontium ions in the oral environment.

The strontium ions in the zeolite can be obtained by impregnating the zeolite in strontium salts such as strontium chloride and strontium nitrate, and the concentration of strontium ions in the zeolite can be adjusted by adjusting the concentration of strontium salts and the number of impregnation times. Similarly, although the zeolite contains calcium ions, a content of the calcium ions in the zeolite can be adjusted by impregnating the zeolite in calcium salts such as calcium chloride.

Furthermore, in some embodiments, the mass ratio of strontium ions to calcium ions in the zeolite is in a range of 1: 1 to 4: 1, so that both an anti-allergic effect and a hemostatic effect of the toothpaste can be better.

It can be understood that, excepting the friction agent, the toothpaste further includes components such as humectant, thickener, surfactant and essence, for example, xanthan gum, glycerol, sorbitol, sodium lauryl sulfate, and so on, which will be not described in detail herein.

Hereinafter, the toothpaste will be further described by the following specific examples.

### First example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3 HB), 7.05% of zeolite (with a particle size *R₂* of 5 µm, a Brinell hardness *H₂* of 5.4 HB, and a mass fraction of calcium ions of 2%), and 0.07% of titanium dioxide (CI77891).

### Second example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 20 µm, and a Brinell hardness *H₁* of 2.5 HB), 7.05% of zeolite (with a particle size *R₂* of 4 µm, a Brinell hardness *H₂* of 4 HB, and a mass fraction of calcium ions of 2%), and 0.07% of titanium dioxide (CI77891).

### Third example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 40 µm, and a Brinell hardness *H₁* of 3.5 HB), 7.05% of zeolite (with a particle size *R₂* of 2 µm, a Brinell hardness *H₂* of 6 HB, and a mass fraction of calcium ions of 2%), and 0.07% of titanium dioxide (CI77891).

### Fourth example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 35 µm, and a Brinell hardness *H₁* of 2.8 HB), 7.05% of zeolite (with a particle size *R₂* of 3 µm, a Brinell hardness *H₂* of 5 HB, and a mass fraction of calcium ions of 2%), and 0.07% of titanium dioxide (CI77891).

### Fifth example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 40 µm, and a Brinell hardness *H₁* of 3.2 HB), 7.05% of zeolite (with a particle size *R₂* of 8 µm, a Brinell hardness *H₂* of 4.16 HB, and a mass fraction of calcium ions of 2%), and 0.07% of titanium dioxide (CI77891).

### Sixth embodiment

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 25 µm, a Brinell hardness *H₁* of 3 HB), 7.05% of zeolite (with a particle size *R₂* of 2 µm, a Brinell hardness *H₂* of 4.5 HB, and a mass fraction of calcium ions of 2%), and 0.07% of titanium dioxide (CI77891).

### First comparative example

By a mass fraction, the toothpaste in the present example was mainly composed of 0.28% of xanthan gum, 15.52% of water, 58.16% of sorbitol, 3.52% of polyethylene glycol -400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 18.33% of silicon dioxide (with a particle size *R₁* of 30 µm, and a Brinell hardness *H₁* of 3 HB), and 0.07% of titanium dioxide (CI77891).

### Second comparative example

By a mass fraction, the toothpaste in the present example was mainly composed of 0.28% of xanthan gum, 15.52% of water, 58.16% of sorbitol, 3.52% of polyethylene glycol -400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 18.33% of zeolite (with a particle size *R₂* of 5 µm, a Brinell hardness *H₂* of 5.4 HB, and a mass fraction of calcium ions of 2%), and 0.07% of titanium dioxide (CI77891).

### Third comparative example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 14.28% of silicon dioxide (with a particle size *R₁* of 30 µm, and a Brinell hardness *H₁* of 3 HB), 4.05% of zeolite (with a particle size *R₂* of 5 µm, a Brinell hardness *H₂* of 5.4 HB, and a mass fraction of calcium ions of 2%), and 0.07% of titanium dioxide (CI77891).

### Fourth comparative example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 4.28% of silicon dioxide (with a particle size *R₁* of 30 µm, and a Brinell hardness *H₁* of 3 HB), 14.05% of zeolite (with a particle size *R₂* of 5 µm, a Brinell hardness *H₂* of 5.4 HB, and a mass fraction of calcium ions of 2%), and 0.07% of titanium dioxide (CI77891).

### Fifth comparative example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3 HB), 7.05% of zeolite (with a particle size *R₂* of 10 µm, a Brinell hardness *H₂* of 5.4 HB, and a mass fraction of calcium ions of 2%), and 0.07% of titanium dioxide (CI77891).

### Sixth comparative example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3 HB), 7.05% of zeolite (with a particle size *R₂* of 1 µm, a Brinell hardness *H₂* of 5.4 HB, and a mass fraction of calcium ions of 2%), and 0.07% of titanium dioxide (CI77891).

### Seventh comparative example

By a mass fraction, the toothpaste in the present example was mainly composed of 0.28% of xanthan gum, 15.52% of water, 58.16% of sorbitol, 3.52% of polyethylene glycol -400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 20 µm, and a Brinell hardness *H₁* of 3 HB), 7.05% of zeolite (with a particle size *R₂* of 5 µm, a Brinell hardness *H₂* of 5.4 HB, and a mass fraction of calcium ions of 2%), and 0.07% of titanium dioxide (CI77891).

### Eighth comparative example

By a mass fraction, the toothpaste in the present example was mainly composed of 0.28% of xanthan gum, 15.52% of water, 58.16% of sorbitol, 3.52% of polyethylene glycol -400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 2.5 HB), 7.05% of zeolite (with a particle size *R₂* of 5 µm, a Brinell hardness *H₂* of 5.4 HB, and a mass fraction of calcium ions of 2%), and 0.07% of titanium dioxide (CI77891).

### Ninth comparative example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3.5 HB), 7.05% of zeolite (with a particle size *R₂* of 5 µm, a Brinell hardness *H₂* of 4 HB, and a mass fraction of calcium ions of 2%), and 0.07% of titanium dioxide (CI77891).

The toothpastes of the first example to the sixth example and the toothpastes of the first to ninth comparative examples were subjected to performance comparison. The cleaning (whitening) effect was compared by using Lobene stain area index, in which Lobene stain area index was compared exogenous colored tooth patients before using the toothpastes with that after using the toothpastes 8 weeks. Hard particles were detected with reference to the GB/T 8372 -2017 standard (method in section 5.7), such that damage of the toothpastes to the teeth was reflected. The hemostatic effect was detected by using in vitro coagulation time. Results were shown in Table 1.

**Table 1**

| | Lobene stain area index | | Whether a slide included scratch | Coagulation time |
|---|---|---|---|---|
| | Before using a toothpaste | Using a toothpaste after 8 weeks | | |
| First example | 1.47 | 0.87 | No | 181 s |
| Second example | 1.47 | 0.86 | No | 183 s |
| Third example | 1.48 | 0.89 | No | 182 s |
| Fourth example | 1.47 | 0.88 | No | 177 s |
| Fifth example | 1.49 | 0.87 | No | 182 s |
| Sixth example | 1.45 | 0.87 | No | 180 s |
| First comparative example | 1.46 | 1.12 | No | 30 minutes, no solidification |
| Second comparative example | 1.47 | 0.89 | Yes | 155 s |
| Third comparative example | 1.48 | 1.07 | No | 212 s |
| Fourth comparative example | 1.47 | 0.91 | Yes | 175 s |
| Fifth comparative example | 1.49 | 1.02 | Yes | 199 s |
| Sixth comparative example | 1.48 | 1.03 | Yes | 197 s |
| Seventh comparative example | 1.48 | 1.01 | No | 191 s |
| Eighth comparative example | 1.46 | 1.04 | Yes | 196 s |
| Ninth comparative example | 1.47 | 1.02 | No | 195 s |

It can be seen from Table 1 that the silicon dioxide and the zeolite are compounded to serve as friction agents for the toothpaste, and the particle sizes, the Brinell hardness and the contents of the silicon dioxide and the zeolite are regulated and controlled for synergy, so that the cleaning effect can be ensured, the teeth can be prevented from being damaged due to the transition friction, and the hemostatic function is achieved.

### Seventh example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 12.72% of silicon dioxide R₁ (with a particle size *R₁* of 30 µm, and a Brinell hardness *H₁* of 3 HB), 5.61% of zeolite (with a particle size *R₂* of 5 µm, a Brinell hardness *H₂* of 5.4 HB, and a mass fraction of calcium ions of 2%), and 0.07% of titanium dioxide (CI77891).

### Eighth example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 9.41% of silicon dioxide (with a particle size *R₁* of 30 µm, and a Brinell hardness *H₁* of 3 HB), 8.92% of zeolite (with a particle size *R₂* of 5 µm, a Brinell hardness *H₂* of 5.4 HB, and a mass fraction of calcium ions of 2%), and 0.07% of titanium dioxide (CI77891).

### Ninth example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 7.8% of silicon dioxide (with a particle size *R₁* of 30 µm, and a Brinell hardness *H₁* of 3 HB), 10.53% of zeolite (with a particle size *R₂* of 5 µm, a Brinell hardness *H₂* of 5.4 HB, and a mass fraction of calcium ions of 2%), and 0.07% of titanium dioxide (CI77891).

### Tenth example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 6.92% of silicon dioxide (with a particle size *R₁* of 30 µm, and a Brinell hardness *H₁* of 3 HB), 11.41% of zeolite (with a particle size *R₂* of 5 µm, a Brinell hardness *H₂* of 5.4 HB, and a mass fraction of calcium ions of 2%), and 0.07% of titanium dioxide (CI77891).

### Eleventh example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 5.15% of silicon dioxide (with a particle size *R₁* of 30 µm, and a Brinell hardness *H₁* of 3 HB), 13.18% of zeolite (with a particle size *R₂* of 5 µm, a Brinell hardness *H₂* of 5.4 HB, and a mass fraction of calcium ions of 2%), and 0.07% of titanium dioxide (CI77891).

Performances of the seventh example to the eleventh example are compared, the method is similar to the above method for the performance comparison, a result thereof is shown in Table 2.

**Table 2**

| | Lobene stain area index | | Whether a slide included scratch | Coagulation time |
|---|---|---|---|---|
| | Before using a toothpaste | Using a toothpaste after 8 weeks | | |
| Seventh example | 1.46 | 0.88 | No | 183 s |
| Eighth example | 1.48 | 0.85 | No | 181 s |
| Ninth example | 1.46 | 0.81 | No | 178 s |
| Tenth example | 1.49 | 0.83 | No | 177 s |
| Eleventh example | 1.47 | 0.82 | No | 175 s |

As shown in Table 2, when a mass proportion of the zeolite in the toothpaste is greater than that of the silicon dioxide in the toothpaste, an effect of the toothpaste is better.

### Twelfth example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3 HB), 7.05% of zeolite (with a particle size *R₂* of 5 µm, a Brinell hardness *H₂* of 5.4 HB, a mass fraction of calcium ions of 0.5%, and a mass fraction of strontium ions of 0.2%), and 0.07% of titanium dioxide (CI77891).

### Thirteenth example

By a mass fraction, the toothpaste in the present example was mainly composed of 0.28% of xanthan gum, 15.52% of water, 58.16% of sorbitol, 3.52% of polyethylene glycol -400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3 HB), 7.05% of zeolite (with a particle size *R₂* of 5 µm, a Brinell hardness *H₂* of 5.4 HB, a mass fraction of calcium ions of 1%, and a mass fraction of strontium ions of 0.5%), and 0.07% of titanium dioxide (CI77891).

### Fourteenth example

By a mass fraction, the toothpaste in the present example was mainly composed of 0.28% of xanthan gum, 15.52% of water, 58.16% of sorbitol, 3.52% of polyethylene glycol -400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3 HB), 7.05% of zeolite (with a particle size *R₂* of 5 µm, a Brinell hardness *H₂* of 5.4 HB, a mass fraction of calcium ions of 1%, and a mass fraction of strontium ions of 1.5%), and 0.07% of titanium dioxide (CI77891).

### Fifteenth example

By a mass fraction, the toothpaste in the present example was mainly composed of 0.28% of xanthan gum, 15.52% of water, 58.16% of sorbitol, 3.52% of polyethylene glycol -400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3 HB), 7.05% of zeolite (with a particle size *R₂* of 5 µm, a Brinell hardness *H₂* of 5.4 HB, a mass fraction of calcium ions of 0.5%, and a mass fraction of strontium ions of 1.5%), and 0.07% of titanium dioxide (CI77891).

### Sixteenth example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 5 µm, and a Brinell hardness *H₁* of 3 HB), 7.05% of zeolite (with a particle size *R₂* of 5 µm, a Brinell hardness *H₂* of 5.4 HB, a mass fraction of calcium ions of 2%, and a mass fraction of strontium ions of 2%), and 0.07% of titanium dioxide (CI77891).

### Seventeenth example

By a mass fraction, the toothpaste in the present example was mainly composed of 0.28% of xanthan gum, 15.52% of water, 58.16% of sorbitol, 3.52% of polyethylene glycol -400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 35 µm, and a Brinell hardness *H₁* of 3 HB), 7.05% of zeolite (with a particle size *R₂* of 5 µm, a Brinell hardness *H₂* of 5.4 HB, a mass fraction of calcium ions of 2%, and a mass fraction of strontium ions of 2%), and 0.07% of titanium dioxide (CI77891).

### Eighteenth example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 11.28% of silicon dioxide (with a particle size *R₁* of 30 µm, and a Brinell hardness *H₁* of 3 HB), 7.05% of zeolite (with a particle size *R₂* of 5 µm, a Brinell hardness *H₂* of 4 HB, a mass fraction of calcium ions of 2%, and a mass fraction of strontium ions of 2%), and 0.07% of titanium dioxide (CI77891).

The toothpastes in the comparative first example 1 and the comparative second example and the toothpastes in the twelfth example to the eighteenth example were tested for tubule sealing performance, and a test of coagulation time is performed by the method of YY/T 1829 -2022, a result thereof was shown in Table 3.

**Table 3**

| | Sealing rate of dental tubules | Coagulation time |
|---|---|---|
| First comparative example | 0 | 30 minutes without solidification |
| Second comparative example | 0.03% | 155s |
| Twelfth example | 34% | 178s |
| Thirteenth example | 35% | 180s |
| Fourteenth example | 41% | 171s |
| Fifteenth example | 43% | 172s |
| Sixteenth example | 42% | 172s |
| Seventeenth example | 37% | 176s |
| Eighteenth example | 38% | 177s |

It can be seen from Table 3 that after the zeolite being loaded with the strontium ions, the toothpaste has functions of sealing tubules and mineralizing dentin, thereby improving the anti-allergic ability of the teeth. The hemostatic effect and the anti-allergic ability of the toothpaste can be further improved by optimizing the concentration of strontium ions and calcium ions and optimizing the particle sizes and the Brinell hardness of the silicon dioxide and the zeolite.

### Tenth comparative example

By a mass fraction, the toothpaste in the present example was mainly composed of 0.28% of xanthan gum, 15.52% of water, 58.16% of sorbitol, 3.52% of polyethylene glycol 400, 0.70% of glycerol, 0.42% of carboxymethylcellulose sodium, 0.35% of sodium pyrophosphate, 0.10% of sucralose, 0.08% of sodium benzoate, 0.99% of sodium dodecyl sulfate, 0.78% of essence, 0.70% of zinc citrate, 18.33% of zeolite (with a particle size *R₂* of 5 µm, and a Brinell hardness *H₂* of 4 HB, a mass fraction of calcium ions of 2%, and a mass fraction of strontium ions of 2%), and 0.07% of titanium dioxide (CI77891).

A test of a taste of the toothpaste were performed on the toothpastes of the sixth example and the tenth comparative example, and a test method was as follows: fifty volunteers were selected into two groups, and each of two groups included twenty-five volunteers, and the toothpastes of the sixteenth example and the toothpastes of the tenth comparative example were used in the two groups, respectively, so as to evaluate a taste, and results thereof were shown as follows: in the group that the volunteers used the toothpastes of the sixth example, the volunteers showed great taste, while in the group that the volunteers used the toothpastes of the tenth comparative example, eight volunteers showed general taste, and seventeen volunteers showed poor taste. Therefore, in the friction agent of the present application, the zeolite can dynamically and continuously release the strontium ions, such that the concentration of the strontium ions in the oral environment is in a relatively stable state, a problem of poor taste caused by too high concentration of the strontium ions is avoided, and the taste of the toothpaste is improved.

The various technical features of the above embodiments can be combined in any way. In order to make the description concise, not all possible combinations of the various technical features in the above embodiments have been described. However, as long as there is no contradiction in the combination of these technical features, they should be considered within the scope of the specification.

The above embodiments only express several embodiments of the present application, and their descriptions are more specific and detailed, but should not be understood as limiting the scope of the disclosure. It should be pointed out that for ordinary skill in the art, several modifications and improvements can be made without departing from the concept of the disclosure, which are within the scope of protection of the disclosure. Therefore, the scope of protection of the present application should be based on the attached claims.

## Claims

1. A toothpaste, **characterized by** comprising 5% to 20% of silicon dioxide and 5% to 15% of zeolite by a mass fraction, wherein an average particle size of the silicon dioxide is defined as *R₁*, a Brinell hardness of the silicon dioxide is defined as *H₁*, an average particle size of the zeolite is defined as *R₂*, and a Brinell hardness of the zeolite is defined as *H₂*, the average particle size *R₁* of the silicon dioxide meets the following relationship: 20 µm ≤ *R₁* ≤ 40 µm, the average particle size *R₂* of the zeolite meets the following relationship: 1 µm ≤ *R₂* ≤ 10 µm, the average particle size *R₁* of the silicon dioxide and the average particle size *R₂* of the zeolite meet the following relationship: 5 ≤ *R₁* / *R₂* ≤ 20, the Brinell hardness *H₁* of the silicon dioxide meets the following relationship: 2.5 HB ≤ *H₁* ≤ 3.5 HB, the Brinell hardness *H₂* of the zeolite meets the following relationship: 4.0 HB ≤ *H₂* ≤ 6HB, and the Brinell hardness *H₁* of the silicon dioxide and the Brinell hardness *H₂* of the zeolite meet the following relationship: 1.3 ≤ *H₂* / *H₁* ≤ 2.

2. The toothpaste of claim 1, wherein the Brinell hardness *H₂* meets the following relationship: 4.0 HB ≤ *H₂* ≤ 5.5 HB.

3. The toothpaste of claim 1, wherein the mass fraction of the silicon dioxide in the toothpaste is less than or equal to that of the zeolite in the toothpaste.

4. The toothpaste of claim 3, wherein a mass ratio of the silicon dioxide to the zeolite is in a range of 1: 1 to 1: 3.

5. The toothpaste of claim 1, wherein a specific surface area of the zeolite is greater than or equal to 50 m²/g; and/or, a pore size of the zeolite is greater than or equal to 0.5 nm.

6. The toothpaste of any one of claims 1 to 5, wherein by a mass fraction of calcium ions in the zeolite is in a range of 0.5% to 3%.

7. The toothpaste of any one of claims 1 to 5, wherein strontium ions are loaded on the zeolite.

8. The toothpaste of claim 7, wherein the average particle size *R₁* of the silicon dioxide meets the following relationship: 20 µm ≤ *R₁* ≤ 30 µm, the average particle size *R₂* of the zeolite meets the following relationship: 5 µm ≤ *R₂* ≤ 10 µm, and the Brinell hardness *H₁* of the silicon dioxide and the Brinell hardness *H₂* of the zeolite meet the following relationship: 1.5 ≤ *H₂* / *H₁* ≤ 2.

9. The toothpaste of claim 7, wherein a mass fraction of the strontium ions in the zeolite is in a range of 0.2% to 2.0%.

10. The toothpaste of claim 9, wherein a mass ratio of the strontium ions in the zeolite to the calcium ion in the zeolite is in a range of 1: 1 to 4: 1.
